## Europäisches Patentamt
## European Patent Office
## Office européen des brevets

(11) Veröffentlichungsnummer: **0 103 854**
**B1**

(12) # EUROPÄISCHE PATENTSCHRIFT

(45) Veröffentlichungstag der Patentschrift:
**10.05.89**

(21) Anmeldenummer: **83109115.2**

(22) Anmeldetag: **15.09.83**

(51) Int. Cl.⁴: **B 01 F 17/34,** B 01 F 17/00,
A 61 K 7/48, A 61 K 9/10

(54) Emulgatorkombinationen für Emulsionen vom Typ Wasser-in-Öl sowie deren Verwendung.

(30) Priorität: **20.09.82 DE 3234786**

(43) Veröffentlichungstag der Anmeldung:
**28.03.84 Patentblatt 84/13**

(45) Bekanntmachung des Hinweises auf die Patenterteilung:
**10.05.89 Patentblatt 89/19**

(84) Benannte Vertragsstaaten:
**AT BE CH DE FR GB IT LI NL SE**

(56) Entgegenhaltungen:
DE-A-2 241 016
FR-A-2 132 130
GB-A-2 002 652

(73) Patentinhaber: **Henkel Kommanditgesellschaft auf Aktien, Postfach 1100 Henkelstrasse 67, D-4000 Düsseldorf- Holthausen (DE)**

(72) Erfinder: **Ansmann, Achim, Fichtestrasse 19, D-4010 Hilden (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft Emulgatorkombinationen aus Gemischen von Einfach- und Mehrfachestern höherer Fettsäuren mit Glycerin und mikrokristallinen Wachsen so wie deren Verwendung.

Emulgatoren zur Herstellung und Stabilisierung von Emulsionen des Typs Wasser-in-Öl (W/O) enthalten nicht nur heute meist durch chemische Synthese erzeugte amphiphile Substanzen wie z. B. partielle Ester von Oligo- oder Polyalkoholen oder Salze höhermolekularer Fettsäuren mit 2- und 3-wertigen Kationen (G. Proserpio und M. Sutti, Parf. u. Kosm. 61, 135 (1980); L.J. Murphy und F. Baiocchi, Cosm. and Toil. 95, 43 (1980)) oder primäre, sekundäre oder tertiäre o-Phosphorsäureester (W. Skrypzak, A.K. Reng und J.M. Quack, Parf. u. Kosm. 60, 317 (1979)), sondern auch emulgierend wirkende Substanzen natürlicher Herkunft wie Bienenwachs, Lecithine oder Sterine tierischen bzw. pflanzlichen Ursprungs. Während die Verwendung von Emulgatoren auf Basis tierischer Sterine, und hier insbesondere des Lanolins und seiner Derivate, im Bereich der kosmetischen Industrie zurückgeht, weil Wollfett und seine Derivate unter Umstanden Allergien hervorrufen und außerdem den damit zubereiteten Cremes einen starken Eigengeruch verleihen, der oft auch durch Parfümierung nicht ganz überdeckt werden kann (vgl. DG-PS-2 241 016) ergeben sich auch beim Einsatz von Sterinenpflanzlichen Ursprungs Nachteile, wie z. B. der sehr hohe Schmelzpunkt pflanzlicher Sterine (135 - 145°C) und relativ schlechtes Ölbindevermögen der mit ihnen hergestellten Emulsionen.

Letzterer Nachteil fällt besonders dann ins Gewicht, wenn mit derartigen emulgierenden Substanzen hergestellte Emulsionen, z. B. Cremes, thermischen oder mechanischen Belastungen ausgesetzt werden; ein Ausölen schon bei niedriger Belastung und nach kurzer Zeit ist die Folge.

Cremeförmige Wasser-in-Öl-Emulsionen, die als Emulgatorbestandteile Sterine tierischen oder pflanzlichen Ursprungs und Vaseline enthalten, sind zudem dadurch gekennzeichnet, daß sie relativ kompakt sind und stark fetten. Die Ansprüche des Marktes hinsichtlich Konsistenz und Fettvermögen derartiger W/O-Cremes laufen dem jedoch diametral entgegen: weiche und schwach fettende Präparate haben in den letzten Jahren erheblich an Bedeutung gewonnen.

Alle genannten Nachteile lassen sich für die gewünschten weichen und schwachfettenden W/O-Emulsionen vermeiden, wenn die erfindungsgemäßen Kombinationen Emulgator/Wachs eingesetzt werden.

Demgemäß betrifft die Erfindung Emulgatorkombinationen aus Fettsäurepartialglyceriden und mikrokristallinen Wachsen sowie deren Verwendung.

Es wurde nun gefunden, daß Kombinationen Emulgator/Wachs aus Fettsäurepartialglyceriden und mikrokristallinen Wachsen vom Typ Ozokerit mit mindestens 45 % verzweigten Kohlenwasserstoffen zu Emulsionen, z. B. Cremes, führen, die die vorstehend geschilderten Nachteile nicht aufweisen. Bei Verwendung dieser Kombinationen kann sowohl auf Sterine als auch auf Vaseline verzichtet werden, ohne Verluste an Stabilität und Ölbindevermögen in Kauf nehmen zu müssen.

Es wurde im Gegenteil überraschenderweise gefunden, daß die nach dem erfindungsgemäßen Verfahren mit den neuen Emulgatorkombinationen hergestellten weichen bis sehr weichen W/O-Cremes eine erheblich verbesserte Stabilität und höhere Ölbindefähigkeit besitzen.

Als Fettsäurepartialglyceride werden erfindungsgemäß Gemische aus Fettsäuremono-, di- und triglyceriden von Fettsäuren mit 12 bis 18 Kohlenstoffatomen verwendet. Vorteilhaft ist die Verwendung von Partialglyceriden auf Basis von ungesättigten Fettsäuren, also von Fettsäureschnitten, die Ölsäure, Linolsäure, Palmitoleinsäure oder Ricinolsäure enthalten. Ebenfalls gut geeignet sind Partialglyceride auf Basis von Isostearinsäure oder diese enthaltenden Fettsäuregemischen. Besonders geeignet sind Fettsäurepartialglyceride, deren Fettsäurezusammensetzung überwiegend aus Palmitinsäure, Stearinsäure und Ölsäure besteht und deren Ölsäureanteil mindestens 40 Gew.-% ausmacht.

Die Fettsäurepartialglyceride werden normalerweise durch Umesterung von Triglyceriden, z. B. von natürlichen Fetten und Ölen, mit Glycerin oder durch Veresterung geeigneter Fettsäureschnitte mit Glycerin hergestellt. Durch die Wahl der Molverhältnisse von Triglyceriden bzw. Fettsäuren zu Glycerin und durch die Reaktionsbedingungen und die Art der Aufarbeitung läßt sich das Verhältnis von Einfach- zu Mehrfachestern des Glycerins in weiten Grenzen variieren.

Für die erfindungsgemäßen Emulgatorkombinationen sind solche Fettsäurepartialglyceride geeignet, die einen Gehalt von mindestens 20 Gew.-% Monoglycerid und von höchstens 20 Gew.-% Triglycerid aufweisen. Fettsäurepartialglyceride mit einem Gehalt von 45 - 50 Gew.-% Monoglycerid, ca. 40 Gew.-% Diglycerid, ca. 10 Gew.-% Triglycerid der jeweils eingesetzten Fettsäuremischung sowie bis zu maximal 5 Gew.-% an freiem Glycerin werden bevorzugt.

Bei den in den erfindungsgemäßen Emulgatorkombinationen eingesetzten mikrokristallinen Wachsen handelt es sich um feinkristalline Mikrowachse, bevorzugt vom Typ Ozokerit, die mindestens zu 45 Gew.-% verzweigt sind und einen Erstarrungspunkt von 65 - 75°C bei einer Penetration von 15- 30 (bei 25°C) aufweisen. Ein geeignetes Handelsprodukt ist Lunacera®M (L.W. Fuller)

Die Komponenten Fettsäureglyceridgemisch und mikrokristallines Wachs werden im Gewichtsverhältnis 90 : 10 bis 16 : 84 zur Herstellung von Wasser-in-Öl-Emulsionen eingesetzt, besonders günstig ist ein Mischungsverhältnis von 75 : 25 bis 35 : 65 für die Emulgierung der meisten handelsüblichen kosmetischen Ölkomponenten, wie z. B. flüssiger Fettsäureester, pflanzlicher Öle und Paraffinöle.

Zur Herstellung von W/O-Emulsionen, z .B. von Hautcremes werden die erfindungsgemäßen Emulgator-Wachskombinationen in Mengen von 1 bis 10 Gew.-%, vorzugsweise von 2 bis 5 Gew.-% eingesetzt.

Besonders vorteilhaft wirkt sich die Verwendung der erfindinngsgemäßen Emulgatorkombinationen auf die

2

Konsistenz von mit ihnen hergestellten W/O-Cremes aus. Bei entsprechender Rezeptur lassen sich weiche bis sehr weiche Cremes herstellen, deren Viskosität nur ca. 150 000 mPas beträgt. Demgegenüber weisen Cremes, die unter Verwendung von Sterinen als Emulgatorbestandteil und Vaseline (z. B. gemäß DE-PS-2 241 016) hergestellt wurden, eine Viskosität von 400 000 mPas auf.

Ein weiterer Vorteil der erfindungsgemäßen Emulgator/Wachs-Kombinationen ist, daß sie den mit ihnen hergestellten W/O-Cremes ein überraschend hohes Ölbindevermögen und überdurchschnittliche Stabilität gegenüber thermischer und mechanischer Belastung verleihen. So halten W/O-Cremes nach den erfindungsgemäßen Rezepturen einer Temperaturbelastung auf 45°C über 12 kochen stand, bevor sie beginnen auszuzuölen, während Cremes mit Sterinen als Emulgatorbestandteil, z. B. gemäß DE-PS-2 241 016, schon nach ca. 2 Wochen beginnen, auszuölen.

Die nachfolgenden Beispiele sollen den Gegenstand der Erfindung weiter erläutern.

**Beispiele**

Im folgenden werden Beispiele für Wasser-in-Öl-Cremes unter Verwendung der erfindungsgemäßen Emulgatorkombinationen angegeben.

Als mikrokristallines Wachs wurde Lunacera®M (L.W. Fuller) verwendet.

Als Fettsäurepartialglyceride wurden die folgenden Handelsprodukte eingesetzt:

Arlacel®186   (Atlas Chemie)
Tegin®ISO   (Goldschmidt)
Cutina®GMS   (Henkel KGaA)

| Bestandteile | Beispiel | | | | |
|---|---|---|---|---|---|
| | 1 | 2 | 3 | 4 | 5 |
| Fettphase | | | | | |
| Isopropylmyristat | - | 3,0 | 5,0 | - | 2,0 |
| Paraffinöl perliquidum | 20,0 | 17,0 | 18,0 | 20,0 | 18,0 |
| Zinkstearat | 2,0 | - | 3,0 | 2,0 | - |
| Magnesiumstearat | - | 2,0 | - | - | 3,0 |
| Bienenwachs | - | 1,0 | - | - | 0,5 |
| Lunacera®M | 1,0 | 1,0 | 2,0 | 2,0 | 2,5 |
| Arlacel®186 | 3,0 | 2,0 | - | - | 1,0 |
| Tegin®ISO | - | - | 2,0 | - | - |
| Cutina®GMS | - | - | - | 2,5 | - |
| Wasserphase | | | | | |
| Glycerin | 3,0 | 4,0 | 5,0 | 3,0 | 3,0 |
| Magnesiumsulfat | 0,4 | 0,4 | 0,5 | 0,5 | 0,5 |
| Konservierungsmittel* | 0,2 | 0,2 | 0,2 | 0,2 | 0,2 |
| Wasser | ad 100 | ad 100 | ad 100 | ad 100 | ad 100 |
| Parfüm | 0,3 | 0,4 | 0,3 | 0,5 | 0,2 |

* (p-Hydroxybenzoesäuremethylester)

**Patentansprüche**

1. Emulgatorkombinationen für Emulsionen vom Typ Wasser-in-Öl, dadurch gekennzeichnet, daß sie aus Gemischen von Einfach- und Mehrfachestern von Fettsäuren mit 12 bis 18 Kohlenstoffatomen mit Glycerin und mikrokristallinen Wachsen bestehen.

2. Emulgatorkombinationen nach Anspruch 1, dadurch gekennzeichnet, daß als Fettsäuren Ölsäure, Linolsäure, Ricinolsäure, Isostearinsäure oder deren Gemische mit gesättigten linearen Fettsäuren enthalten sind.

3. Emulgatorkombinationen nach Anspruch 1 und 2, dadurch gekennzeichnet, daß als Fettsäuren Gemische aus Palmitinsäure, Stearinsäure und Ölsäure enthalten sind.

4. Emulgatorkonbinationen nach Ansprüchen 1 - 3, dadurch gekennzeichnet, daß das Estergemisch mindestens 20 % Monoglycerid und maximal 20 % Triglycerid enthält.

5. Emulgatorkombinationen nach Ansprüchen 1 - 4, dadurch gekennzeichnet, daß das Estergemisch bevorzugt 50 % Monoglyceride, 40 % Diglyceride und 10 % Triglyceride enthält.

6. Emulgatorkombinationen nach Ansprüchen 1 - 5, dadurch gekennzeichnet, daß man als Wachsbestandteil der Kombination mikrokristalline Wachse vom Typ Ozokerit mit mindestens 45 % verzweigten Kohlenwasserstoffen verwendet.

3

7. Emulgatorkombinationen nach Ansprüchen 1 - 6, dadurch gekennzeichnet, daß Fettsäureglyceridgemisch und mikrokristalline Wachse im Verhältnis von 90 : 10 bis 16 : 84 Gew.-% enthalten sind.

8. Emulgatorkombinationen nach Ansprüchen 1 - 7, dadurch gekennzeichnet, daß das bevorzugte Verhältnis Fettsäureglyceridgemisch zu mikrokristallinen Wachsen 75 : 25 bis 35 : 65 Gew.-% beträgt.

9. Verwendung der Emulgatorkombinationen nach Ansprüchen 1 - 8 in W/O-Emulsionen, insbesondere Hautcremes, dadurch gekennzeichnet, daß man einen Anteil von 1 bis 10 Gew.-%, vorzugsweise 2 - 5 Gew.-% der Emulgatorkombination, bezogen auf die gesamte Emulsion, einsetzt.

## Claims

1. Emulsifier combinations for emulsions of the water-in-oil type, characterized in that they consist of mixtures of mono- and poly-esters of fatty acids containing 12 to 18 carbon atoms with glycerol and microcrystalline waxes.

2. Emulsifier combinations as claimed in claim 1, characterized in that they contain oleic acid, linoleic acid, ricinoleic acid, isostearic acid or mixtures thereof with saturated linear fatty acids as fatty acids.

3. Emulsifier combinations as claimed in claims 1 and 2, characterized in that they contain mixtures of palmitic acid, stearic acid and oleic acid as fatty acids.

4. Emulsifier combinations as claimed in claims 1 to 3, characterized in that the ester mixture contains at least 20 % monoglyceride and at most 20 % triglyceride.

5. Emulsifier combinations as claimed in claims 1 to 4, characterized in that the ester mixture preferably contains 50 % monoglycerides, 40 % diglycerides and 10 % triglycerides.

6. Emulsifier combinations as claimed in claims 1 to 5, characterized in that microcrystalline waxes of the ozocerite type containing at least 45 % branched hydrocarbons are used as the wax component of the combination.

7. Emulsifier combinations as claimed in claims 1 to 6, characterized in that the fatty acid glyceride mixture and microcrystalline waxes are present in a ratio of from 90 : 10 to 16 : 84 % by weight.

8. Emulsifier combinations as claimed in claims 1 to 7, characterized in that the preferred ratio of fatty acid glyceride mixture to microcrystalline waxes is from 75 : 25 to 35 : 65 % by weight.

9. The use of the emulsifier combinations claimed in claims 1 to 8 in w/o emulsions, particularly hair creams, characterized in that the emulsifier combination is used in a quantity of 1 to 10 % by weight and preferably in a quantity of 2 to 5 % by weight, based on the emulsion as a whole.

## Revendications

1. Associations d'émulsifiants pour la réalisation d'émulsions du type eau dans l'huile, caractérisées par le fait qu'elles sont constituées de mélanges de monoesters et de polyesters de glycérol et d'acides gras comportant 12 à 18 atomes de carbone avec des cires microcristallines.

2. Associations d'émulsifiants selon la revendication 1, caractérisées par le fait qu'elles comportent comme acides gras l'acide oléique, l'acide linoléique, l'acide ricinoléique, l'acide isostéarique ou des mélanges de ces acides avec des acides gras linéaires saturés.

3. Associations d'émulsifiants selon les revendications 1 et 2, caractérisées par le fait qu'elles comportent comme acides gras des mélanges d'acide palmitique, d'acide stéarique et d'acide oléique.

4. Associations d'émulsifiants selon les revendications 1 à 3, caractérisées par le fait que le mélange d'esters comporte au minimum 20 % de monoglycérides et au maximum 20 % de triglycérides.

5. Associations d'émulsifiants selon les revendications 1 à 4, caractérisées par le fait que le mélange d'esters comporte de préférence 50 % de monoglycérides, 40 % de diglycérides et 10 % de triglycérides

6. Associations d'émulsifiants selon les revendications 1 à 5, caractérisées par le fait que les cires faisant partie de l'association sont des cires microcristallines du type ozokérite, comportant au minimum 45 % d'hydrocarbures ramifiés.

7. Associations d'émulsifiants selon les revendications 1 à 6, caractérisées par le fait que le mélange de glycérides d'acide gras et les cires microcristallines y sont contenus dans des proportions, exprimées en % en poids, dont le rapport est compris dans l'intervalle de 90 : 10 à 16 : 84.

8. Associations d'émulsifiants selon les revendications 1 à 7, caractérisées par le fait que la proportion préférée du mélange de glycérides d'acides gras et celle des cires microcristallines sont entre elles dans un rapport compris dans l'intervalle de 75 : 25 à 35 : 65, lesdites proportions étant exprimées en % en poids.

9. Utilisation des associations d'émulsifiants selon les revendications 1 à 8 dans des émulsions du type eau dans l'huile, en particulier des crèmes dermatologiques, caractérisée par l'emploi d'une fraction de 1 à 10 % en poids - de préférence 2 à 5 % en poids - des associations d'émulsifiants, par rapport à la quantité totale d'émulsion.